# EUROPEAN PATENT APPLICATION

(11) **EP 3 150 214 A1**
(43) Date of publication of application: **05.04.2017**
(21) Application number: 15425078.1
(22) Date of filing: 01.10.2015
(51) Int. Cl.: A61K 36/9066, A61K 36/9068, A61P 29/00, A61P 25/02, A61P 19/02

(54) **COMPOSITIONS CONTAINING EXTRACTS OF CURCUMA SPP AND ZINGIBER OFFICINALE WHICH ARE USEFUL TO REDUCE PERIPHERAL INFLAMMATION AND PAIN**

(71) Applicant: INDENA S.p.A., 20139 Milano (IT)
(72) Inventor: Togni, Stefano, 20139 Milano (IT); Franceschi, Federico, 20139 Milano (IT); Malandrino, Salvatore, 20139 Milano (IT)
(74) Representative: Bianchetti Bracco Minoja S.r.l.

(57) **Abstract**

Disclosed are compositions containing an extract of *Curcuma spp,* optionally as curcumin in the form of a complex with phospholipids, and a lipophilic extract of *Zingiber officifiale,* for use in the treatment of osteoarthritis.

## Description

### Summary of the invention

The present invention relates to compositions containing an extract of *Curcuma spp* (turmeric), optionally as curcumin in the form of a complex with phospholipids, and a lipophilic extract of *Zingiber officinale,* which are useful in the treatment of osteoarthritis, especially in patients unable to tolerate long-term treatment with NSAIDs or steroids.

### State of the art

Osteoarthritis is one of the major causes of physical disability in the elderly. As well as worsening the quality of life of millions of people worldwide, it also causes financial loss to both families and the community, as it gives rise to very high healthcare costs.

Conventional osteoarthritis treatments involve symptomatic treatment with analgesics and therapeutic treatment with non-steroidal anti-inflammatory drugs, which are well-known to induce side effects that are often serious, leading to discontinuance of the treatment. Similar considerations apply to rheumatoid arthritis. For these reasons, researchers have focused on phytopharmaceuticals and natural plant-based remedies in general.

Turmeric roots and rhizomes have been used as spices since time immemorial in India, and are also used in many industrialised countries nowadays. Their active ingredient is curcumin, the yellow compound that constitutes the main ingredient of curry and other traditional Indian dishes. The uses described in popular medicine include the treatment of indigestion, flatulence, diarrhoea, and above all inflammatory states and joint pain after long-term treatment. Many of these traditional uses have been confirmed by bioassays *in vitro* or in pharmacology.

Curcumin is one of the molecules that is being most widely investigated nowadays from the biochemical standpoint, and in recent years, in addition to numerous publications of doubtful scientific value, double-blind controlled clinical trials have also been conducted according to the dictates of modern clinical pharmacology. Preliminary clinical trials demonstrated the low bioavailability of the molecule, which is unstable at intestinal pH (half-life at pH 7 < 10 min), and its low absorption when administered orally, which said factors limit many of the applications based on tests *in vitro.* Plasma concentrations of 50 ng have been reported after administration of 12 g of compound, also measuring the secondary metabolites, glucuronides and sulphates.

Like many polyphenols, curcumin is poorly soluble in water and fats. The Applicant has therefore devised a process (disclosed in WO2007101551) that involves complexing curcumin with phospholipids to improve its bioavailability. Curcumin complexed with phospholipids provides better systemic absorption, which allows its use for human administration.

Clinical trials conducted on patients with osteoarthritis have demonstrated that long-term treatment with curcumin complexed with phospholipids (Belcaro et al., Alternative Medicine Review, 4, 338, 2010) gives results which are favourable, but still insufficient due to poor pain reduction, which obliges the patient to take NSAIDs.

Significant improvements were only obtained after 8 months treatment, and many patients had to use NSAIDs or other analgesics to relieve the pain.

*Zingiber officinale* roots and rhizomes treated in various ways are used as spices in India and China. The uses described in popular medicine include the treatment of indigestion, flatulence, diarrhoea, coughing, and other correlated disorders. Extracts of said plant have been mainly considered for their anti-nausea effect. However, the findings of the controlled clinical trials are contradictory, and the US Pharmacopoeia recommends a complete review of the properties attributed to the plant due to the lack of convincing documentation. Some of the conflicting data are partly attributable to the instability of the active ingredients in the extracts normally used.

The extract used in the present invention is a lipophilic extract, stabilised and prepared with carbon dioxide under well-defined supercritical conditions.

### Description of the invention

It has now surprisingly been found that compositions containing an extract of *Curcuma spp,* optionally as curcumin in the form of a complex with phospholipids, and a lipophilic extract of *Zingiber officinale,* have a potent analgesic and anti-inflammatory effect, greater than that found when extracts of *Curcuma spp* and *Zingiber officinale* are used separately.

Particularly good results have been obtained with compositions containing curcumin complexed with phospholipids and lipophilic extracts of *Zingiber officinale.*

The extract of the roots and rhizomes of *Zingiber officinale* can be obtained by extraction from the roots and rhizomes with lipophilic solvents such as ethyl acetate, hexane and aliphatic esters, or preferably by extraction with carbon dioxide under supercritical conditions, which guarantees the stability of the active ingredients and prevents the formation of undesirable compounds or other inactive products of oxidation.

The *Zingiber officinalis* extract used in the present invention, prepared by extraction from the rhizomes with carbon dioxide under supercritical conditions, is characterised by a content of 20 to 35% gingerols, preferably 25%.

The compositions according to the invention can be administered orally. According to a preferred aspect thereof, the compositions for oral administration will contain curcumin complexed with phospholipids in quantities ranging from 100 to 1000 mg, and *Zingiber officinale* extract in quantities ranging from 20 to 200 mg per oral unit dose.

According to a particularly preferred aspect, the compositions for oral administration will contain 500 mg of curcumin complexed with phospholipids and 50 mg of *Zingiber officinale* extract per oral unit dose.

The lipophilic extract of *Zingiber officinale* is preferably prepared by extraction from the roots and rhizomes of the plant at pressures ranging between 230 and 260 bars, preferably 235 bars, at a temperature ranging between 40 and 60°C, preferably 50°C, for a time ranging between 1 and 10 hours, preferably seven hours; the extract is collected in the condenser and dehydrated in inert gas dissolved in n-hexane or heptane containing a lipophilic antioxidant, preferably ascorbyl palmitate or tocopherol, and concentrated under vacuum at a temperature not exceeding 40°C.

The *Zingiber officinale* rhizome extract typically contains 20 to 35% gingerol together with other terpenes.

The oral compositions according to the invention preferably take the form of soft gelatin capsules or rigid capsules or tablets after microencapsulation and/or absorption of oils.

Said compositions can be prepared by conventional methods, as described in Remington's Pharmaceutical Handbook, Mack Publishing Co., N.Y., USA, using suitable excipients.

The following examples further illustrate the invention.

### EXAMPLE 1 - SOFT GELATIN CAPSULES

| | |
|---|---|
| *Curcuma spp* extract | 200 mg |
| *Zingiber officinalis* lipophilic extract | 25 mg |
| Soya lecithin | 10 mg |
| Palmitoyl ascorbate | 20 mg |
| *Oeyaothera biennis* lipophilic extract | 300 mg |

### EXAMPLE 2 - CELLULOSE CAPSULES FOR OILS

| | |
|---|---|
| Phospholipid complex of curcumin | 500 mg |
| *Zingiber officinalis* lipophilic extract | 50 mg |
| Soya lecithin | 10 mg |

### EXAMPLE 3 - TABLETS

| | |
|---|---|
| Phospholipid complex of curcumin | 500mg |
| *Zingiber officinalis* lipophilic extract | 50 mg |
| Soya lecithin | 10 mg |
| Palmitoyl ascorbate | 20 mg |
| Microcrystalline cellulose | 300 mg |

### Pharmacological experimentation

A study was conducted on 100 volunteers suffering from hip osteoarthritis to evaluate whether the combination of curcumin and *Zingiber officinalis* could provide additional benefits, with a possible synergistic effect. The clinical end-points (WOMAC score, Karnofsky Performance Scale Index and treadmill walking performance) were evaluated for the single agents and for the combination of the two vs. a placebo. An improvement in the clinical end-points was observed for both curcumin and *Zingiber officinale* compared with the placebo group.

Surprisingly, the combination of the two ingredients induced a more than additive beneficial effect, demonstrating the novel synergistic effect of the association, with a faster onset of action and more pronounced efficacy than would be expected from the sum of the effects of the single ingredients. This, coupled with excellent tolerability, demonstrates that the composition according to the invention can constitute an invaluable tool for long-term complementary management of pain and inflammation, as in osteoarthritis, inducing a rapid and readily measurable beneficial effect.

### Patients and methods

### Patients

100 patients with grade I or II hip osteoarthritis, confirmed by X-ray analysis, were enrolled in the study. The patients were allowed to self-medicate with NSAIDs during the 8-month trial. Four groups of subjects with symptomatic osteoarthritis were defined: Group A was managed using curcumin at the dose of 1000 mg/day; Group B was managed using an extract of *Zingiber officinale* equivalent to the dose of 100 mg/day. Group C received a combination of curcumin and *Zingiber officinale* at the same doses as reported above for the single ingredients. The last group received a corresponding placebo. To ensure clinical homogeneity, the main location of the osteoarthritis in these subjects, and the source of most of their signs and symptoms, was in one or both hips.

Primary osteoarthritis in one or both hips was diagnosed by X-ray investigation. The subjects had mild to moderate pain not adequately or completely controlled with anti-inflammatory drugs. They had to be able to perform the treadmill walking test and to understand all the questions in the WOMAC questionnaire.

### Evaluation of functional impairment

The Karnofsky Performance Scale Index was used to classify the patients in terms of functional impairment. The Karnofsky Performance Scale Index can be used to compare the effectiveness of different treatments and to assess the prognosis in individual patients. The lower the Karnofsky score, the worse the patient's functional impairment.

### Evaluation of signs/symptoms of osteoarthritis

The questionnaire developed by the Western Ontario and McMaster Universities was applied to describe and rate the symptoms of OA. The questionnaire produces scores for the various symptoms of OA (WOMAC scores). The status of the OA signs/symptoms was evaluated by the investigator together with the patient on inclusion, and after 1 month, 4 months and 8 months treatment.

### Evaluation of physical performance

The patients were trained to perform the treadmill test in two tutorial tests. On inclusion and after 1 month, 4 months and 8 months treatment, the patient's performance was evaluated with the treadmill test at a speed of 3 km/h and an incline of 10%. The total pain-free distance was recorded.

### Statistical analysis

The variations in the results (i.e. walking distance) were considered parametric. Non-parametric observations (e.g. the WOMAC score) were evaluated with analysis of variance (ANOVA with the Bonferroni correction). Considering the possible intra-individual and inter-individual data variations, at least 40 completing subjects were needed for this type of study (at least 40 subjects who completed the entire follow-up period in each group). This numerical value was established, on the basis of our previous study, with a view to eliminating spontaneous variability and time variations. OA and its related signs/symptoms, even under conditions of relative stability, may have a spontaneous degree of variability associated with periods of improvement. Clinical variations in signs/symptoms, walking distance, oedema and the Karnofsky scale may be due to various clinical and non-clinical (i.e. environmental or climatic) factors, including individual, psychological and drug-related elements.

### Results

At the beginning of this study, the treatment groups (3 groups of 25 patients each) and the placebo group (25 patients) did not differ in terms of age or male-to-female ratio, or in terms of the Karnofsky Performance Scale Index, overall WOMAC score and performance on the treadmill test.

The results of the Karnofsky Performance Scale Index are presented in Table 1; both curcumin and *Zingiber officinale* significantly improved the patients' Karnofsky Scale scores, but the combination of said ingredients provided earlier results which clearly suggest a synergistic effect, whereas there were no significant improvements in the control group.

**Table 1. Variations in the Karnofsky scale (median and range)**

| | Inclusion | 1 month | 4 months | 8 months |
|---|---|---|---|---|
| Curcumin | 73 | 77 | 80* | 81* |
| *Zingiber officinale* | 74 | 76 | 81* | 81* |
| Combination | 74 | 93* | 95* | 96* |
| Placebo | 75 | 77 | 73 | 75 |

| | | | | |
|---|---|---|---|---|
| *p<0.05 | | | | |

The results of the evaluation, based on WOMAC scores during the 8-month study, are presented in Table 2. The pain scores fell significantly (p<0.05) after only one month's administration of curcumin or Zingiber, while the difference improved further, demonstrating a synergistic action between the two ingredients, in the combination group, whereas there were no significant effects in the control group.

**Table 2. Variation in mean scores after 8 months treatment**

| | Curcumin | *Zingiber officinale* | Curcumin + *Zingiber officinale* | Control | | | |
|---|---|---|---|---|---|---|---|
| WOMA C items | R** | 1 month | R** | 1 month | R** | 1 month | R** |
| Pain | 16 | 13 | 16 | 14 | 16 | 7* | 16 |
| Stiffness | 7 | 5 | 6 | 5 | 8 | 2* | 8 |
| Physical function s | 55 | 45 | 55 | 52 | 57 | 20* | 53 |

| | | | | | | | |
|---|---|---|---|---|---|---|---|
| *p<0.05 R** = recruitment | | | | | | | |

Table 3 shows the results of the physical exercise (treadmill) tests (median). The treadmill (at the speed of 3 km/hour, with a 10% incline) showed a dramatic improvement in the initial distance (p<0.05) after only 1 month in the combination group, which was much higher than in the single-ingredient groups.

**Table 3. Results of physical exercise test (median). The treadmill test was performed with a treadmill moving at the speed of 3 km/hour, with a 10% incline**

| | Curcumin | *Zingiber officinale* | Curcumin + *Zingiber officinale* | Control | | | |
|---|---|---|---|---|---|---|---|
| | R** | 1 month | R** | 1 month | R** | 1 month | R** |
| Distance | 77 | 100* | 82 | 88 | 81 | 340* | 82 |

| | | | | | | | |
|---|---|---|---|---|---|---|---|
| *p<0.5 R** = recruitment | | | | | | | |

## Claims

1. Compositions containing *Zingiber officinale* lipophilic extract and *Curcuma spp* extract for use in the treatment of peripheral pain, superficial and deep inflammatory and pain states, and pain associated with muscle spasms.

2. Compositions according to claim 1 containing curcumin in the form of a complex with phospholipids.

3. Compositions according to claim 1 for the treatment of osteoarthritis.

4. Compositions according to claim 1 wherein the *Zingiber officinale* extract is obtained by extraction from the roots and/or rhizomes of the plant with supercritical carbon dioxide.
